# EUROPÄISCHE PATENTSCHRIFT

(11) **EP 2 195 000 B1**
(45) Veröffentlichungstag und Bekanntmachung des Hinweises auf die Patenterteilung: **25.09.2019**
(21) Anmeldenummer: 08787461.6
(22) Anmeldetag: 26.08.2008
(51) Int. Cl.: A61K 36/87, A61P 17/00

(54) **SPRÜHFÄHIGE ZUSAMMENSETZUNG ENTHALTEND EXTRAKT AUS ROTEM WEINLAUB**
SPRAYABLE COMPOSITION CONTAINING RED VINE LEAF EXTRACT
COMPOSITION PULVÉRISABLE CONTENANT DE L'EXTRAIT DE FEUILLE DE VIGNE ROUGE

(30) Priorität: 31.08.2007 DE 102007041556
(43) Veröffentlichungstag der Anmeldung: 16.06.2010
(73) Patentinhaber: Sanofi-Aventis Deutschland GmbH, 65929 Frankfurt am Main (DE)
(72) Erfinder: BUSZELLO, Katrin, 55216 Ingelheim am Rhein (DE); FREICHEL, Oliver Ludwig, 55216 Ingelheim am Rhein (DE); LANGER, Martin, 55216 Ingelheim am Rhein (DE); PLOHMANN, Bernd, 55216 Ingelheim am Rhein (DE)
(74) Vertreter: Weickmann & Weickmann PartmbB
(86) Internationale Anmeldenummer: PCT/EP2008/061110
(87) Internationale Veröffentlichungsnummer: WO 2009/027382

(56) Entgegenhaltungen:
- WO-A1-2004/058280
- DE-U1-202007 006 374
- US-A1- 2005 053 560
- US-A1- 2006 110 415
- ANONYMOUS: "Rotes Weinlaub" CMD Naturkosmetik INTERNET ARTICLE 2007, XP002540133 Gefunden im Internet: URL:http://www.cmd-naturkosmetik.de/Rotes- Weinlaub-2007.pdf> [gefunden am 2009-08-05]

## Beschreibung

### Erfindungshintergrund

Viele Menschen, vor allem Frauen, neigen zur Venenschwäche, die auch als chronische venöse Insuffizienz (CVI) bekannt ist. Menschen mit CVI leiden an schweren, geschwollenen Beinen, insbesondere bei warmer Witterung oder nach langem Sitzen oder Stehen. Diese Symptome schränken die Funktion der Beine erheblich ein, was wiederum negative Auswirkungen auf die Mobilität und Aktivität und allgemein auf das Wohlbefinden der Betroffenen hat. Nach der etablierten Kategorisierung nach Widmer wird die klinisch relevante CVI in drei Stadien eingeteilt: Stadium 1: Reversible Ödeme, Corona phlebectatica (dunkelblaue Hautvenenveränderungen am med. und lat. Fussrand), perimalleoläre Kölbchenvenen; Stadium 2: persistierende Ödeme, Hämosiderose und Purpura der Haut im Unterschenkelbereich, Dermatosklerosen und Lipodermatosklerose, Atrophie blanche, Stauungsekzem, zyanotische Hautfarbe; Stadium 3: Ulzera Cruris.

Extrakte von rotem Weinlaub der Gattung *vitis vinifera* sind seit langem bekannt und verbreitet. In der traditionellen oder Volksmedizin werden entsprechende Präparate schon seit langer Zeit angewendet zur Behandlung einer großen Palette von Leiden und Beschwerden. So wird z.B. in Frankreich, wie in der Pharmacopée francaise nachzulesen ist, Rotweinlaubextrakt als Venentonikum für die Behandlung verschiedener Venenleiden verwendet.

Moderne Präparate auf der Basis von Rotweinlaub sind beispielsweise die von Boehringer Ingelheim unter der Marke Antistax® angebotenen Kapseln und Tabletten zur Behandlung von chronisch venöser Insuffizienz. Diese enthalten einen Rotweinlaubextrakt mit hohem Gehalt an Flavonoiden, welche entsprechend den Erkenntnissen medizinischer Gutachten eine entscheidende Rolle bei der Abdichtung von Venen spielen sowie eine entzündungshemmende Wirkung aufweisen. Die vorgenannten Kapseln und Tabletten sind ausschließlich zur oralen Anwendung gedacht.

Weiterhin marktgängig ist eine Antistax® Salbe zur topischen Anwendung auf der Haut. Diese Salbe enthält auch Rotweinlaubextrakt. Die Salbe muß jedoch per Hand auf die betreffenden Hautstellen aufgetragen werden, wodurch sich für den Anwender einerseits Probleme durch die mit Salbe behafteten Handflächen ergeben und andererseits die Auftragung der Salbe oft inhomogen erfolgt.

Daher stellt sich die Aufgabe, eine Rotweinlaubextrakt enthaltende Zusammensetzung bereitzustellen, die gleichmäßig und ohne Kontakt mit den Handflächen des Anwenders auf die Haut aufgetragen werden kann.

### Zusammenfassung der Erfindung

Die hier beschriebene Erfindung löst obiges Problem durch Bereitstellung einer sprühfähigen Zusammensetzung enthaltend Rotweinlaubextrakt, vorzugsweise aus *vitis vinifera L.* Diese kann mit geeigneten Sprühvorrichtungen auf die Haut gesprüht werden, so daß eine relativ gleichmäßige Auftragung der Zusammensetzung erreicht wird, ohne daß diese mit den Händen des Anwenders in direkten Kontakt gelangt.

Die vorliegende Erfindung betrifft eine sprühfähige Zusammensetzung bestehend aus Rotweinlaubextrakt, Wasser, einem mit Wasser unter erhöhtem Druck mischbaren Treibmittel sowie einem oder mehreren weiteren Inhaltsstoffen, ausgewählt aus der Gruppe bestehend aus Lösungsmitteln, Hauptpflegesubstanzen und Hilfsstoffen, wobei das Treibmittel Dimethylether umfaßt.

Aus EP 1 581 239 ist bekannt, daß wäßriger Rotweinlaubextrakt sich positiv auf die Mikrozirkulation in den Kapillaren der unteren Extremitäten auswirkt. Dies wurde durch Anwendung von Laser Doppler Flowmetry gezeigt (vgl. auch Tulevski II, Ubbink DT, Jacobs MJHM. Red and green laser Doppler compared with capillary microscopy to assess skin microcirculation in the feet of healthy subjects. Microvasc Res 1999;58(2):83-88; und Bollinger A, Jäger K, Jünger M, Seifert H. The vascular laboratory: advances in non-invasive techniques. World J Surg 1988;12:724-731).

DE 20 2007 006374 offenbart venentherapeutische Zubereitungen zur topischen Applikation umfassend als Wirkstoffe pflanzliche Extrakte von Gingko biloba, Hamamelis virginiana und Vitis vinifera sowie zumindest ein Hydroxymethyl- und/oder Hydroxyethyl-Derivat von Rutin bzw. Rutoside, vorzugsweise Troxerutin oder Oxerutin, die vorteilhaften synergistik zusammenwirken. Jedoch besteht die besonders wirkungsvolle Form der Zubereitung aus einem hydrophilen Gel.

US 2006/0110415 offenbart Zusammensetzungen - in Form eines Sprays - mit verbesserter tiefer Hautpenetration, die gegebenenfalls Rotweinlaubextrakte zur Verbesserung der Mikrozirkulation enthalten.

Eine besonders bevorzugte Ausführungsform der vorliegenden Erfindung ermöglicht eine gleichmäßigere Auftragung von Rotweinlaubextrakt auf die Haut mit schnellerer und optimaler Permeation der wirksamen flavonoidartigen Inhaltsstoffe, so daß eine gleichmäßigere und effektive Wirkung in den oberen Hautschichten (z.B. im Corium) resultiert.

### Detaillierte Beschreibung der Erfindung

Die sprühfähige Zusammensetzung nach der vorliegenden Erfindung enthält als Hauptbestandteil Rotweinlaubextrakt, typischerweise in einer Konzentration von 0.1 Gewichts-% bis 15 Gewichts-%, vorzugsweise von 0.3 Gewichts-% bis 10 Gewichts-% und besonders bevorzugt von 0.5 Gewichts-% bis 8 Gewichts-%, jeweils bezogen auf den Trockenextrakt und die Gesamtmasse der Zusammensetzung. Dieser wird vorzugsweise durch Extraktion aus den Blättern von *vitis viniferaL.* gewonnen, vorzugsweise zu einem Zeitpunkt, zu dem die Flavonoidkonzentration in den Blättern ein Optimum erreicht hat. Dies ist etwa zur Zeit der Traubenernte erreicht. Die Extraktion wird vorzugsweise als Flüssigextraktion durchgeführt, das heißt mit einem flüssigen Auszugsmittel. Als Auszugsmittel kommen alle für die Herstellung Nahrungsmittel bzw. Pharmazeutika akzeptablen Auszugsmittel in Betracht. Bevorzugt sind Ethanol und Wasser sowie deren Gemische. Nachstehend ist ein bevorzugtes Verfahren zur Herstellung des Rotweinlaubextraktes zur Verwendung im Rahmen der vorliegenden Erfindung beschrieben, es umfaßt folgende Schritte:
(a) Sammeln von Rotweinblättern zu einem Zeitpunkt, zu dem der Gehalt an Flavonoiden ein Optimum erreicht hat;
(b) Trocknen und Zerkleinern der Blätter;
(c) Zerschneiden der Blätter in Stücke;
(d) Extraktion der zerschnittenen Blätter mit Wasser oder Ethanol oder deren Gemischen bei erhöhter Temperatur für 6 bis 10 Stunden;
(e) optional Konzentrieren des erhaltenen Extraktes.

Beim Zerschneiden der Blätter in Schritt (c) fallen vorzugsweise Stücke mit einer Größe im Bereich von 5 bis 10 mm an. Die Extraktion in Schritt (d) erfolgt vorzugsweise bei erhöhter Temperatur, vorzugsweise im Bereich von 60°C bis 80°C. Das Konzentrieren des Extraktes in Schritt (e) erfolgt vorzugsweise durch Feuchtigkeitsentzug im Vakuum und kann bis zur Trockne erfolgen.

Im Rahmen der vorliegenden Erfindung wird vorzugsweise Rotweinlaubextrakt eingesetzt, der Flavonoide im Bereich von 1 bis 20 Gewichts-%, vorzugsweise 2 bis 10 Gewichts-% enthält, in Bezug auf die Trockenmasse des Extraktes.

Neben dem Rotweinlaubextrakt enthält die sprühfähige Zusammensetzung der vorliegenden Erfindung Wasser und ein Treibmittel optional noch weitere Inhaltsstoffe. Bevorzugt sind Hautpflegesubstanzen und Hilfsstoffe.

Das Treibmittel umfasst Dimethylether, der aufgrund seiner Lösungseigenschaften im wässrigen Milieu eine kurzfristige Übersättigung der Inhaltsstoffe, insbesondere der Flavonoide, auf der Haut beim Sprühen ermöglicht und damit auch eine schnelle Permeation in die oberen Hautschichten. Die thermodynamische Aktivität, definiert als Konzentration der Wirksubstanz im Vehikel zur Ihrer Sättigungslöslichkeit, wird durch dieses Prinzip größer als 1, so dass der aufgenommene Wirkstoffanteil (maximaler Flux) größer als bei einem herkömmlichen hydrophilen Gelen ist.

In bevorzugten Ausführungsformen der vorliegenden Erfindung umfaßt das Treibmittel
Dimethylether in einer Menge von typischerweise wenigstens 1 Gewichts-%, vorzugsweise wenigstens 5 Gewichts-%, bevorzugt wenigstens 10 Gewichts-%, besonders bevorzugt wenigstens 20 Gewichts-%, jeweils bezogen auf die Gesamtmasse der Zusammensetzung.

Dimethylether verdunstet aufgrund seines hohen Dampfdruckes schnell auf der Haut des Anwenders, die durch die so entstehende Verdunstungskälte gekühlt wird. Dieser Kühlungseffekt trägt neben dem entzündungshemmenden und venenabdichtenden Effekt des Rotweinlaubextraktes kurzfristig auch zur Linderung der CVI-Beschwerden bei.

"Lösungsmittel" im Rahmen der vorliegenden Erfindung bezeichnet alle kosmetisch und pharmazeutisch akzeptablen Mittel, die geeignet sind, Bestandteile der sprühfähigen Zusammensetzung zu lösen. Bevorzugte Lösungsmittel sind Wasser, aliphatische Alkohole wie z.B. Ethanol, Isopropanol, Glykole wie z.B. Glycerol, Propylenglykol oder Fettsäureester, wie z.B. Isopropylmyristat, Ölsaureoleylester sowie hochmolekulare Lösungsmittel, wie z.B. Polytheylenglycole, sowie deren Mischungen. Ein bevorzugtes Lösungsmittel ist Wasser.

"Hautpflegesubstanzen" im Rahmen der vorliegenden Erfindung bezeichnet alle kosmetisch und pharmazeutisch akzeptablen Mittel, die bei direkter Auftragung positive Wirkungen auf das Aussehen und die Gesundheit der menschlichen Haut haben. Bevorzugt sind Hautfeuchteregulatoren wie z.B. Propylenglykol, Harnstoff oder Panthenol, Substanzen, die die Aufnahme von Stoffen durch die Haut verbessern (Penetrationsbeschleuniger), wie z.B. Harnstoff und Propylenglycol, und Stoffe, die die Hautregeneration verbessern, wie z.B. Panthenol, sowie deren Mischungen. Besonders bevorzugte Hautpflegesubstanzen sind Harnstoff und Panthenol. Bevorzugte Penetrationsbeschleuniger sind Harnstoff und Propylenglycol.

"Hilfsstoffe" im Rahmen der vorliegenden Erfindung bezeichnet alle kosmetisch und pharmazeutisch akzeptablen Mittel, die die physikalischen Eigenschaften der sprühfähigen Zusammensetzung oder deren Stabilität optimieren. Bevorzugt sind Stabilisatoren, wie z.B. Aminomethyl-Propanol, Konservierungsstoffe, wie z.B. Methylparaben, Emulgatoren, wie z.B. PEG-40 hydrogenated castor oil, Viskositätsadjustierer wie z.B. Isopropylmyristat und Gelbildner, wie z.B. vernetzte und unvernetzte Polyacrylate, Carboxymethylcellulose Hydroxyethylcellulose, Methylcellulose. Weitere Hilfsstoffe sind Farb- und Duftstoffe. Hilfsstoffe wie Isopropylmyristat tragen u.a. dazu bei, daß die Zusammensetzung besser auf der Haut spreitet, d.h. sich besser verteilt.

Besonders bevorzugte Ausführungsformen der Zusammensetzung nach der vorliegenden Erfindung sind sogenannte Sprühgele. Diese weisen eine Viskosität im Bereich von 15000 bis 70000 mPas, vorzugsweise wenigstens 20000 mPas, besonders bevorzugt wenigstens 50000 mPas und besonders bevorzugt wenigstens 60000 mPas, jeweils bei 20°C, auf und verweilen länger auf der Haut als geringerviskose Zusammensetzungen und können daher auch länger ihre Wirkung auf den beauftragten Hautpartien entfalten.

Die sprühfähige Zusammensetzung kommt erfindungsgemäß in einem Sprühbehälter zum Einsatz, aus dem es durch eine Sprühvorrichtung, typischerweise eine Sprühdüse, auf die Haut des Anwenders gesprüht wird. Geeignete Sprühbehälter mit geeigneten Sprühvorrichtungen sind marktgängig. Besonders geeignete Sprühbehälter für Sprühgele sind die Sprühbehälter von kommerziell erhältlichen Produkten wie Hansaplast Sprühpflaster, Polysport Sprühpflaster, Scholl Gelpflaster, Flint Sprühverband, Hansaplast Aktiv GelPflaster, Compeed Liquid Sprühpflaster oder Softivel Sprühpflaster. Entsprechend der vorliegenden Erfindung steht die Zusammensetzung im Sprühbehälter unter einem Druck im Bereich von 1.5 bis 20 bar, vorzugsweise von 2 bis 15 bar, bevorzugt von 3 bis 12 bar und besonders bevorzugt von 4 bis 8 bar, jeweils bei 20°C, steht.

Eine besonders bevorzugte Ausführungsform der vorliegenden Erfindung ist ein Rotweinlaubextrakt enthaltendes hydrophiles Sprühgel mit unter Druck im wässrigen Milieu löslichen Treibmitteln, umfassend Dimethylether und welches vorzugsweise weiterhin Harnstoff, Propylenglycol, Isopropylmyristat und Panthenol als zusätzliche Hauptbestandteile enthält.

Nachstehend ist eine besonders bevorzugte Ausführungsform der sprühfähigen Zusammensetzung nach der vorliegenden Erfindung aufgeführt:

| | |
|---|---|
| Gereinigtes Wasser | 66.49 Gew.% |
| Dimethyl Ether | 26.00 Gew.% |
| Harnstoff | 2.91 Gew.% |
| 1.2 Propylene glycol | 1.74 Gew.% |
| Flüssigextrakt aus Rotweinlaub, Auszugsmittel: 60% Ethanol in Wasser; 1:1 Mischung mit Auszugsmittel | 1.00 Gew.% |
| Isopropyl Myristate | 0.58 Gew.% |
| D-Panthenol 75 W | 0.44 Gew.% |
| Carbomer 940 (Synthalen K) | 0.21 Gew.% |
| Aminomethyl Propanol (AMP Ultra PC 1000) | 0.20 Gew.% |
| PEG-40 Hydrogenated Castor Oil (Cremophor RH 40) | 0.17 Gew.% |
| Methylparaben | 0.07 Gew.% |
| Parfüm | 0.17 Gew.% |

## Patentansprüche

1. Sprühfähige Zusammensetzung bestehend aus Rotweinlaubextrakt, Wasser, einem mit Wasser unter erhöhtem Druck mischbaren Treibmittel sowie einem oder mehreren weiteren Inhaltsstoffen, ausgewählt aus der Gruppe bestehend aus Lösungsmitteln, Hauptpflegesubstanzen und Hilfsstoffen, wobei das Treibmittel Dimethylether umfaßt.

2. Zusammensetzung nach Anspruch 1, wobei das Auszugsmittel für die Gewinnung des Rotweinlaubextraktes aus der Gruppe bestehend aus Wasser, Ethanol und deren Gemischen ausgewählt wurde.

3. Zusammensetzung nach einem der vorstehenden Ansprüche, wobei der Rotweinlaubextrakt durch Extraktion aus den Blättern der Spezies *vitis vinifera L.* gewonnen wurde.

4. Zusammensetzung nach einem der vorstehenden Ansprüche, wobei der Rotweinlaubextrakt nach einem Verfahren hergestellt wurde, das folgende Schritte umfaßt:
(a) Sammeln von Rotweinblättern zu einem Zeitpunkt, zu dem der Gehalt an Flavonoiden ein Optimum erreicht hat;
(b) Trocknen und Zerkleinern der Blätter;
(c) Zerschneiden der Blätter in Stücke;
(d) Extraktion der zerschnittenen Blätter mit Wasser oder Ethanol oder deren Gemischen bei erhöhter Temperatur für 6 bis 10 Stunden;
(e) Optional Konzentrieren des erhaltenen Extraktes.

5. Zusammensetzung nach einem der vorstehenden Ansprüche, wobei die Zusammensetzung Rotweinlaubextrakt in einer Konzentration von 0.1 Gewichts-% bis 15 Gewichts-%, vorzugsweise von 0.3 Gewichts-% bis 10 Gewichts-% und besonders bevorzugt von 0.5 Gewichts-% bis 8 Gewichts-%, jeweils bezogen auf den Trockenextrakt und die Gesamtmasse der Zusammensetzung, enthält.

6. Zusammensetzung nach einem der vorstehenden Ansprüche, wobei der Rotweinlaubextrakt Flavonoide im Bereich von 1 bis 20 Gewichts-%, vorzugsweise 2 bis 10 Gewichts-%, in Bezug auf die Trockenmasse des Rotweinlaubextraktes, enthält.

7. Zusammensetzung nach einem der vorstehenden Ansprüche, enthaltend wenigstens 1 Gewichts-%, vorzugsweise wenigstens 5 Gewichts-%, bevorzugt wenigstens 10 Gewichts-%, besonders bevorzugt wenigstens 20 Gewichts-%, des Treibmittels jeweils bezogen auf die Gesamtmasse der Zusammensetzung.

8. Zusammensetzung nach einem der vorstehenden Ansprüche, wobei die Lösungsmittel ausgewählt sind aus Wasser, Ethanol, Isopropanol, Glycerol, Propylenglykol, Fettsäureestern, Ölsäureoleylestern und Polyethylenglykolen sowie deren Mischungen.

9. Zusammensetzung nach einem der vorstehenden Ansprüche, wobei die Hauptpflegesubstanzen ausgewählt sind aus Propylenglykol, Harnstoff, Panthenol und deren Mischungen.

10. Zusammensetzung nach einem der vorstehenden Ansprüche, wobei die Hilfsstoffe ausgewählt sind aus Aminomethyl-Propanol, Methylparaben, PEG-40 hydrogenated castor oil, Isopropylmyristat, vernetzten und unvernetzten Polyacrylaten.

11. Zusammensetzung nach einem der vorstehenden Ansprüche, wobei die Zusammensetzung ein Sprühgel ist mit einer Viskosität im Bereich von 15000 bis 70000 mPas, vorzugsweise wenigstens 20000 mPas, besonders bevorzugt wenigstens 50000 mPas und besonders bevorzugt wenigstens 60000 mPas, jeweils bei 20°C und 1 bar.

12. Sprühbehälter enthaltend die Zusammensetzung nach einem der vorstehenden Ansprüche.

## Claims

1. Sprayable composition consisting of red vine leaf extract, water, a propellant miscible with water under elevated pressure, and one or more further ingredients, selected from the group consisting of solvents, major care substances and auxiliaries, wherein the propellant comprises dimethyl ether.

2. Composition according to Claim 1, wherein the extractant for obtaining the red vine leaf extract has been selected from the group consisting of water, ethanol and mixtures thereof.

3. Composition according to either of the preceding claims, wherein the red vine leaf extract has been obtained by extraction from the leaves of the species *Vitis vinifera L.*

4. Composition according to any of the preceding claims, wherein the red vine leaf extract has been produced by a method which comprises the following steps:
(a) collecting red vine leaves at a point in time at which the flavonoid content has reached an optimum;
(b) drying and comminuting the leaves;
(c) cutting up the leaves into pieces;
(d) extracting the cut-up leaves with water or ethanol or mixtures thereof at elevated temperature for 6 to 10 hours;
(e) optionally concentrating the resultant extract.

5. Composition according to any of the preceding claims, wherein the composition comprises red vine leaf extract in a concentration of 0.1 weight% to 15 weight%, preferably of 0.3 weight% to 10 weight% and more preferably of 0.5 weight% to 8 weight%, based in each case on the dry extract and the total mass of the composition.

6. Composition according to any of the preceding claims, wherein the red vine leaf extract comprises flavonoids in the range from 1 to 20 weight%, preferably 2 to 10 weight%, in relation to the dry mass of the red vine leaf extract.

7. Composition according to any of the preceding claims, comprising at least 1 weight%, preferably at least 5 weight%, more preferably at least 10 weight%, very preferably at least 20 weight% of the propellant, based in each case on the total mass of the composition.

8. Composition according to any of the preceding claims, wherein the solvents are selected from water, ethanol, isopropanol, glycerol, propylene glycol, fatty acid esters, oleic acid oleyl esters and polyethylene glycols and also mixtures thereof.

9. Composition according to any of the preceding claims, wherein the major care substances are selected from propylene glycol, urea, panthenol and mixtures thereof.

10. Composition according to any of the preceding claims, wherein the auxiliaries are selected from aminomethyl-propanol, methylparaben, PEG-40 hydrogenated castor oil, isopropyl myristate, and crosslinked and non-crosslinked polyacrylates.

11. Composition according to any of the preceding claims, wherein the composition is a spray gel having a viscosity in the range from 15 000 to 70 000 mPas, preferably at least 20 000 mPas, more preferably at least 50 000 mPas and very preferably at least 60 000 mPas, in each case at 20°C and 1 bar.

12. Spray receptacle containing the composition according to any of the preceding claims.

## Revendications

1. Composition pulvérisable constituée d'extrait de feuille de vigne rouge, d'eau, d'un agent propulseur miscible à l'eau sous pression élevée ainsi que d'un ou de plusieurs autre(s) ingrédient(s), choisi(s) dans le groupe constitué par les solvants, les substances de soin principal et les adjuvants, l'agent propulseur comprenant de l'éther diméthylique.

2. Composition selon la revendication 1, l'agent d'extraction pour l'obtention de l'extrait de feuille de vigne rouge ayant été choisi dans le groupe constitué par l'eau, l'éthanol et les mélanges de ceux-ci.

3. Composition selon l'une des revendications précédentes, l'extrait de feuille de vigne rouge ayant été obtenu par extraction des feuilles de l'espèce *vitis vinifera L.*

4. Composition selon l'une des revendications précédentes, l'extrait de feuille de vigne rouge ayant été préparé selon un procédé qui comprend les étapes suivantes :
(a) ramassage de feuilles de vigne rouge à un moment où la teneur en flavonoïdes a atteint un optimum ;
(b) séchage et déchiquetage des feuilles ;
(c) découpage des feuilles en morceaux ;
(d) extraction des feuilles découpées avec de l'eau ou de l'éthanol ou avec des mélanges de ceux-ci à température élevée pendant 6 jusqu'à 10 heures ;
(e) éventuellement, concentration de l'extrait obtenu.

5. Composition selon l'une des revendications précédentes, la composition contenant un extrait de feuille de vigne rouge en une concentration de 0,1% en poids jusqu'à 15% en poids, préférablement de 0,3% en poids jusqu'à 10% en poids et particulièrement préférablement de 0,5% en poids jusqu'à 8% en poids, rapporté à chaque fois à l'extrait sec et à la masse totale de la composition.

6. Composition selon l'une des revendications précédentes, l'extrait de feuille de vigne rouge contenant des flavonoïdes dans la plage de 1 jusqu'à 20% en poids, préférablement de 2 jusqu'à 10% en poids, par rapport à la matière sèche de l'extrait de feuille de vigne rouge.

7. Composition selon l'une des revendications précédentes, contenant au moins 1% en poids, de préférence au moins 5% en poids, préférablement au moins 10% en poids, particulièrement préférablement au moins 20% en poids de l'agent propulseur, rapporté à chaque fois à la masse totale de la composition.

8. Composition selon l'une des revendications précédentes, les solvants étant choisis parmi l'eau, l'éthanol, l'isopropanol, le glycérol, le propylèneglycol, les esters d'acides gras, les esters oléyliques de l'acide oléique et les polyéthylèneglycols ainsi que les mélanges de ceux-ci.

9. Composition selon l'une des revendications précédentes, les substances de soin principal étant choisies parmi le propylèneglycol, l'urée, le panthénol et les mélanges de ceux-ci.

10. Composition selon l'une des revendications précédentes, les adjuvants étant choisis parmi l'aminométhylpropanol, le méthylparabène, le PEG-40-huile de ricin hydrogénée, le myristate d'isopropyle, les polyacrylates réticulés ou non réticulés.

11. Composition selon l'une des revendications précédentes, la composition étant un gel en spray présentant une viscosité dans la plage de 15.000 jusqu'à 70.000 mPa.s, de préférence d'au moins 20.000 mPa.s, particulièrement préférablement d'au moins 50.000 mPa.s, particulièrement préférablement d'au moins 60.000 mPa.s, à chaque fois à 20°C et à 1 bar.

12. Récipient pulvérisateur contenant la composition selon l'une des revendications précédentes.
